# EUROPÄISCHE PATENTSCHRIFT

(11) **EP 3 871 652 B1**
(45) Veröffentlichungstag und Bekanntmachung des Hinweises auf die Patenterteilung: **15.01.2025**
(21) Anmeldenummer: 21158014.7
(22) Anmeldetag: 18.02.2021
(51) Int. Cl.: A61K 8/73, A61K 8/41, A61K 8/86, A61Q 17/00, A61Q 19/10, A61K 8/02, A61K 8/60, A61K 8/44

(54) **WASSERBASIERTES TRÄNKUNGSMITTEL FÜR FEUCHTTÜCHER**
WATER-BASED SATURATION AGENT FOR WET WIPES
AGENT D'IMPRÉGNATION À BASE D'EAU POUR LINGETTES HUMIDES

(30) Priorität: 25.02.2020 DE 102020104943
(43) Veröffentlichungstag der Anmeldung: 01.09.2021
(73) Patentinhaber: Dr. Schumacher GmbH, 34323 Malsfeld (DE)
(72) Erfinder: Schumacher, Sönke, 34323 Malsfeld (DE)
(74) Vertreter: Sandvoß, Stefanie

(56) Entgegenhaltungen:
- EP-A1- 3 494 850
- EP-A2- 2 866 785
- EP-B1- 2 866 785
- WO-A1-2014/120566
- WO-A1-2020/173639

## Beschreibung

Feuchttücher sind bekanntermaßen Vliesstoffe, die mit einer in der Regel flüssigen Zubereitung getränkt sind, die u.a. einen reinigenden und/oder pflegenden Inhaltsstoff enthält. Feuchttücher finden mittlerweile in vielen Bereichen der persönlichen Pflege, der Babypflege, aber auch der Oberflächendesinfektion und -reinigung Anwendung und erfreuen sich sehr hoher Beliebtheit, da sie die Möglichkeit einer schnellen und bequemen Reinigung bzw. Pflege bieten, ohne dass dafür zusätzliches Wasser erforderlich ist. In der Regel zu handlichen Einheiten von ca. 10 bis 200 Tüchern verpackt, zeichnen sich Feuchttücher darüber hinaus auch durch ihre Portabilität aus und sind vor allem auch aufgrund dieser Eigenschaft aus unserer immer mobiler werdenden Gesellschaft nicht mehr wegzudenken.

Feuchttücher, die mit einem Tränkungsmittel getränkt sind, das einen Gehalt an Tensiden aufweist, neigen zu einer starken Schaumentwicklung. Bereits beim Tränken der Tücher in der Produktion kann es selbst bei geringen Konzentrationen an Tensiden zu einer starken Schaumentwicklung kommen, die beim anschließenden Verpacken das Verschweißen der Folien verhindert bzw. in teils erheblichem Ausmaß verzögert.

Des Weiteren wird das Vorhandensein von Schaum in der Verpackung von Feuchttüchern von den Anwendern oftmals als ein Qualitätsmangel empfunden, da das sensorische Empfinden bei der Entnahme der Tücher aus der Packung anders ist als bei Feuchttüchern, die mit einem Tränkungsmittel ohne Schaumbildung getränkt sind. Es besteht daher bei der Herstellung von Feuchttüchern das Bestreben, die Schaumbildung bei der Tränkung von Vliesstoffen zu vermeiden.

Die DE 10 2005 016 687 A1 beschreibt kosmetische Zusammensetzungen, die Dehydroxanthan Gum und mind. 5 Gew.-% Ethanol enthalten und sich durch ein verbessertes Anschäumvermögen auszeichnen sollen. Ethanol wird in diesem Zusammenhang als schaumvermindernd beschrieben. Weiterhin bewirkt Ethanol eine Viskositätsverringerung, weshalb es in Kombination mit einem Verdicker eingesetzt wird. Eine Tränkung von Feuchttüchern mit den beschriebenen Zusammensetzungen wird nicht beschrieben.

Die GB 2 197 789 A beschreibt ein Desinfektionsmittel mit einem hohen Anteil an Formaldehyd, das ein Silikonöl als Entschäumer enthält. Entschäumer auf Silikonbasis haben allerdings den Nachteil, dass sie zum Teil in wässrigen Formulierungen schlecht löslich sind und/oder nach Lagerung bei Raumtemperatur oder leicht erhöhter Temperatur keine ausreichende Entschäumerwirkung mehr aufweisen. Das beschriebene Desinfektionsmittel dient der Reinigung chirurgischer Geräte. Dementsprechend wird das Mittel nicht auf die Haut appliziert und auch nicht als Tränkungsmittel für Feuchttücher verwendet.

Die WO 2015/124303 A1 beschreibt schaumarme agrochemische Zusammensetzungen, die N-Alkylglucamide und einen oder mehrere Entschäumer enthalten. Aufgrund der beabsichtigten agrochemischen Anwendung sind die beschriebenen Zusammensetzungen nicht zur Anwendung auf der Haut vorgesehen und werden dementsprechend auch nicht als Tränkungsmittel für Feuchttücher verwendet bzw. sind nicht dafür geeignet.

Die DE 10 2019 202 723 A1 beschreibt getränkte Tücher, wobei das Tränkungsmittel Biotenside, insbesondere Glycolipide und besonders bevorzugt Rhamnolipide enthält. Durch die Verwendung von Biotensiden soll die von herkömmlichen Tensiden bekannte ausgeprägte Schaumbildung vermieden werden.

Die WO 2014/120566 A1 beschreibt wässrige Zusammensetzungen, die als Tränkungsmittel für Feuchttücher verwendet werden können und einen hohen Anteil an Verdicker enthalten. Das Schäumungsverhalten der Zusammensetzung wird nicht thematisiert.

Die EP 3 494 850 A1 beschreibt ein mit einem Tränkungsmittel getränktes Feuchttuch, wobei das Tränkungsmittel Hydroxyacetophenon als alleiniges Konservierungsmittel enthält. Das Tränkungsmittel ist entweder eine O/W-Emulsion oder eine wässrige Lösung. In der Ausführungsform als O/W-Emulsion enthält das Tränkungsmittel 0,1-0,5 Gew.-% Verdicker, in der Ausführungsform als wässrige Lösung enthält das Tränkungsmittel hingegen gar keinen Verdicker. Ein Tensidsystem, das Betaine und/oder Lauryl Glucosid umfasst, kann optional ebenfalls in dem Tränkungsmittel der D1 enthalten sein.

Die EP 2 866 785 A2 / WO 2014/002019 A2 beschreibt eine O/W-Emulsion als Tränkungsmittel für Feuchttücher, die ein Gemini-Tensid oder einen Phosphatester sowie eine Gummimischung enthält. Die Gummimischung kann Xanthan Gum oder Guar Gum enthalten. Als bevorzugtes Gemini-Tensid wird Dinatrium-ethylen-dicocamid-PEG-15-disulfat genannt.

Die WO 2014/173639 A1 beschreibt Tränkungsmittel für Feuchttücher, die Glucomannan, Xanthan Gum, Carrageen und optional Tenside enthalten und eine Viskosität von 0,8-7,0 Pas aufweisen.

### Aufgabe der Erfindung

Es ist die Aufgabe der vorliegenden Erfindung, ein alternatives Tränkungsmittel für Vliesstoffe sowie ein damit getränktes Feuchttuch bereitzustellen, wobei sich das Tränkungsmittel trotz eines Gehaltes an Tensid durch eine deutlich verringerte und vorzugsweise gänzlich verhinderte Schaumbildung während der Herstellung des Tränkungsmittels und der Tränkung des Vliesstoffs auszeichnet.

### Allgemeine Beschreibung der Erfindung

Die Erfindung löst diese Aufgabe mit den Merkmalen der Ansprüche und insbesondere mit einem Tränkungsmittel für Feuchttücher, das eine wasserbasierte Zusammensetzung ist, die mindestens 90 Gew.-% Wasser, bezogen auf das Gesamtgewicht der Zusammensetzung, sowie zumindest ein Tensid und zumindest einen Verdicker enthält, wobei der Anteil an in der Zusammensetzung enthaltenem Verdicker maximal 0,1 Gew.% beträgt, bezogen auf das Gesamtgewicht der Zusammensetzung, wobei das zumindest eine Tensid aus der Gruppe ausgewählt ist, die aus Alkylpolyglycosiden, Betain-Tensiden, ethoxylierten Tensiden, Sulfo-Tensiden und quarternären Ammoniumverbindungen besteht, und wobei die dynamische Viskosität der Zusammensetzung bei 20 °C maximal 12 mPas, gemessen mit einem DIN-Messsystem 11 gemäß ISO 53019 bei einer Temperatur von 293,15 K und mit einer Umdrehungszahl von 1000 r/min, beträgt.

Unter einem Sulfo-Tensid wird ein Tensid verstanden, das mindestens eine Sulfonsäuregruppe (-SO₃H) aufweist.

Für die Zwecke der Erfindung wird unter einem Feuchttuch ein Vliesstoff verstanden, der mit einer flüssigen oder halbflüssigen Zusammensetzung kontaktiert bzw. getränkt wurde. Neben den klassischen getränkten rechteckigen oder quadratischen Vliesstoffen werden insbesondere auch nicht rechteckige Vliesstoffe, beispielsweise runde oder ovale Pads, die mit einer flüssigen oder halbflüssigen Zusammensetzung kontaktiert bzw. getränkt wurden, für die Zwecke der Erfindung als Feuchttuch bezeichnet.

Das erfindungsgemäße wasserbasierte Tränkungsmittel enthält mindestens 90 Gew.-% Wasser. Es ist jedoch bevorzugt, dass der Anteil an Wasser in der Zusammensetzung noch größer ist, insbesondere > 93 Gew.-%, > 94 Gew.-%, > 95 Gew.-%, > 96 Gew.-%, > 97 Gew.-% oder > 98 Gew.-%, jeweils bezogen auf das Gesamtgewicht der Zusammensetzung.

Erfindungsgemäß enthält das Tränkungsmittel zumindest ein Tensid, das vorzugsweise ein pflanzliches Tensid ist. Der Gehalt an Tensiden ist in herkömmlichen Tränkungsmitteln für deren Neigung zur Schaumbildung beim Tränken von Vliesstoffen verantwortlich. In dem erfindungsgemäßen Tränkungsmittel wird die Schaumbildung beim Tränken von Vliesstoffen jedoch durch einen ebenfalls in dem Tränkungsmittel enthaltenen geringen Gehalt an Verdicker deutlich verringert bzw. sogar ganz vermieden.

Unter einem Verdicker wird für die Zwecke der Erfindung ein hydrophiler Gelbildner verstanden. Erfindungsgemäß ist der Anteil an Verdicker in dem Tränkungsmittel sehr gering und beträgt insbesondere weniger als 0,1 Gew.-%, vorzugsweise weniger als 0,08 Gew.-%, noch bevorzugter weniger als 0,07 Gew.-% und am meisten bevorzugt weniger als 0,06 Gew.-%. Es wurde gefunden, dass es bereits bei den vorgenannten sehr niedrigen Konzentrationen an Verdicker, wie beispielsweise Xanthan Gum und/oder Konjac Gum, in dem Tränkungsmittel sensorisch zu keiner signifikanten Viskositätserhöhung kommt, aber dennoch die Schaumbildung signifikant verringert wird. Dies ist überraschend, da man eigentlich genau das Gegenteil, nämlich eine Stabilisierung des Schaums durch einen Anteil an Verdicker, erwarten würde. Eine stabilisierende Wirkung von Verdickern auf Schaum wird beispielsweise in der US20100055266A1 sowie auch in der US20140287980A1 beschrieben.

Der Anteil an in dem erfindungsgemäßen Tränkungsmittel enthaltenen Tensid beträgt vorzugsweise < 1 Gew.-%, insbesondere < 0,7 Gew.-%, < 0,6 Gew.-% oder < 0,5 Gew.-%, jeweils bezogen auf das Gesamtgewicht der Zusammensetzung.

Gemäß einer Ausführungsform enthält das erfindungsgemäße Tränkungsmittel ein oder mehrere Tenside, die unabhängig voneinander aus der Gruppe ausgewählt sind, die aus Kokosglucosid, Cocamidopropyl-Betain, Caprylyl/Caprylglucosid, Polysorbat 20, Polysorbat 80, PEG-40 hydriertem Rizinusöl, C9-C11-Fettalkoholethoxylat, Natriumlaurylsulfat; Natriumlaurylethersulfat, Benz-alkoniumchlorid und Dodecylammoniumchlorid besteht.

Der in dem erfindungsgemäßen Tränkungsmittel enthaltene zumindest eine Verdicker kann insbesondere aus der Gruppe ausgewählt sein, die aus Konjac Gum, Xanthan Gum, Guar, Cellulose sowie Derivaten von Konjac Gum, Xanthan Gum, Guar und Cellulose besteht. Auch zwei oder mehr in dem erfindungsgemäßen Tränkungsmittel enthaltene Verdicker können unabhängig voneinander aus der vorgenannten Gruppe ausgewählt sein.

Unabhängig davon, ob das erfindungsgemäße Tränkungsmittel einen Verdicker oder zwei Verdicker oder mehrere Verdicker enthält, sind diese insbesondere aus der Gruppe ausgewählt, die aus Konjac Gum, Amorphallus Konjac Root Extract, Xanthan Gum, Dehydroxanthan Gum, Hydroxypropyl Xanthan Gum, Guar, Hydroxypropyl Guar, hydrolisiertem Guar, Carboxymethylhydroxypropyl Guar, Guar Hydroxypropyltrimoniumchlorid, Cellulose, C12-C16 Alkoxyhydroxypropyl-ethylhydroxyethyl-cellulose, C12-C16 Alkyl-PEG-2-hydroxypropylhydroxyethyl-ethylcellulose, Butoxyhydroxypropylcetyl-hydroxyethylcellulose, Carboxymethyl-hydroxyethylcellulose, Carboxymethyl-celluloseacetatbutyrat, Calciumcarboxymethylcelluose, Celluloseacetatpropionatcarboxylat, Cetylhydroxyethylcellulose, Cellulosesuccinat, Cellulose Gum, Croscarmellose, Ethylcellulose, Hydroxybutylmethylcellulose, hydrolisiertem Cellulose Gum, Hydroxyethylcellulose, Hydroxyethyl-ethylcellulose, Hydroxypropyl-methylcellulose, Hydroxypropyl-methylcellulose-stearoxyether, Hydroxypropylcellulose-stearyl-carbamat, Hydroxypropylcellulose, Methyl-Hydroxyethylcellulose, Methyl-ethylcellulose, mikrobieller Cellulose, Methylcellulose, mikrokristalliner Cellulose, oxidierter Cellulose, Natriumstearoxy-PGhydroxyethylcellulosesulfonat, Natriumsalzen oxidierter Cellulose und Natriumcellulosesulfat besteht.

Gemäß einer besonders bevorzugten Ausführungsform enthält das Tränkungsmittel mindestens 90 Gew.-% Wasser, bevorzugt mindestens 93 Gew.-% Wasser und am meisten bevorzugt mindestens 95 Gew.-% Wasser, jeweils bezogen auf das Gesamtgewicht der das Tränkungsmittel bildenden Zusammensetzung, sowie weiterhin zumindest ein Tensid und eine Kombination aus Konjac Gum sowie Xanthan Gum als Verdicker, wobei der Gesamtanteil an Verdicker in der das Tränkungsmittel bildenden Zusammensetzung maximal und insbesondere weniger als 0,1 Gew.% beträgt, bevorzugt weniger als 0,07 Gew.% und besonders bevorzugt weniger als 0,05 Gew.%, jeweils bezogen auf das Gesamtgewicht der Zusammensetzung.

Generell enthält das erfindungsgemäße Tränkungsmittel neben Wasser, einem oder mehreren Tensid(en) und einem oder mehreren Verdicker(n) optional noch weitere Inhaltsstoffe. Dabei kann es in der Auswahl der enthaltenen Inhaltsstoffe für verschiedene Anwendungen angepasst sein, insbesondere für kosmetische Anwendungen oder reinigende Anwendungen, einschließlich der Desinfektion von Haut oder Oberflächen.

Als Tränkungsmittel für kosmetische Anwendungen enthält die Zusammensetzung insbesondere auch einen oder mehrere Inhaltsstoffe, die unabhängig voneinander aus der Gruppe ausgewählt sind, die hautpflegende Wirkstoffe, Feuchthaltemittel, Puffer, Verbindungen mit konservierenden Eigenschaften, Lösungsmittel und Parfum umfasst oder daraus besteht. Als Tränkungsmittel für reinigende und insbesondere desinfizierende Anwendungen enthält die Zusammensetzung bevorzugt auch einen oder mehrere Inhaltsstoffe, die unabhängig voneinander aus der Gruppe ausgewählt sind, die antimikrobielle Wirkstoffe, biozide Wirkstoffe, Feuchthaltemittel, hautpflegende Wirkstoffe, Puffer, Verbindungen mit konservierenden Eigenschaften, Lösungsmittel und Alkohole umfasst oder daraus besteht.

Gemäß einer Ausführungsform enthält die das erfindungsgemäße Tränkungsmittel bildende Zusammensetzung Feuchthaltemittel, die aus der Gruppe ausgewählt sind, die aus Glycerin und Glucose besteht. Der Gesamtanteil an Feuchthaltemittel beträgt dabei vorzugsweise 0,5-1,0 Gew.-%, bezogen auf das Gesamtgewicht der Zusammensetzung.

Gemäß einer Ausführungsform enthält die das erfindungsgemäße Tränkungsmittel bildende Zusammensetzung Konservierungsmittel, die aus der Gruppe ausgewählt sind, die aus Natriumbenzoat, Kaliumsorbat und Ethanol besteht.

Gemäß einer Ausführungsform enthält die das erfindungsgemäße Tränkungsmittel bildende Zusammensetzung einen oder mehrere hautpflegende Wirkstoffe, der bzw. die aus der Gruppe ausgewählt sind, die aus Prunus amygdalus dulcis seed extract, Ethylhexylglycerin, Allantoin, Chamomilla Recutita Flower Extract, Milchsäure und Aloe Barbadensis Leaf Juice besteht.

Die Erfindung betrifft weiterhin auch ein Feuchttuch, das einen mit einem erfindungsgemäßen Tränkungsmittel getränkten Vliesstoff umfasst oder daraus besteht.

Gemäß einer Ausführungsform besteht der in einem erfindungsgemäßen Feuchttuch enthaltene Vliesstoff aus Viskosefasern und/oder Lyocell-Fasern und/oder PET-Fasern, d.h. er kann entweder zu 100 % aus Viskosefasern, Lyocell-Fasern oder PET-Fasern bestehen, oder aus Mischfasern dieser bestehen.

Optional enthält der Vliesstoff auch Mikrofasern und/oder Baumwoll-Fasern, beispielsweise zu einem Anteil von jeweils bis zu 20 Gew.-%, insbesondere von 0,1 - 5,0 Gew.-%.

Gemäß einer bevorzugten Ausführungsform besteht der in dem erfindungsgemäßen Feuchttuch enthaltene Vliesstoff aus Viskosefasern und/oder Lyocell-Fasern und/oder PET-Fasern und/oder Hanffasern und/oder Cellulosefasern und/oder Flachsfasern, sowie optional zusätzlich aus 0,1 - 20,0 Gew.-% Mikrofasern und/oder 0,1 - 20,0 Gew.-% Baumwoll-Fasern.

Besonders bevorzugt umfasst oder besteht der Vliesstoff eines erfindungsgemäßen Feuchttuches aus 30 - 70 Gew.-% Viskosefasern und 30 - 70 Gew.-% PET-Fasern sowie optional 0,1 - 5,0 Gew.-% Mikrofasern und/oder 0,1 - 30,0 Gew.-% Baumwoll-Fasern.

In Ausführungsformen, in denen der Vliesstoff weder Lyocell-Fasern, noch Mikrofasern oder Baumwoll-Fasern enthält, sondern ausschließlich aus Viskosefasern und PET-Fasern besteht, kann der Anteil von Viskosefasern zu PET-Fasern stufenlos variieren, beispielsweise von 1 Gew.-% Viskosefasern / 99 Gew.-% PET-Fasern zu 99 Gew.-% Viskosefasern / 1 Gew.-% PET-Fasern.

Die erfindungsgemäße Eignung des Tränkungsmittels zur nicht schäumenden Herstellung von Feuchttüchern konnte dabei auf allen vorgenannten Vliesstoffen bzw. Vliesstoffzusammensetzungen gezeigt werden.

Die Erfindung betrifft weiterhin auch ein Verfahren zur Herstellung eines Feuchttuches, umfassend das Aufbringen eines Tränkungsmittels auf einen Vliesstoff, wobei das Tränkungsmittel eine wasserbasierte Zusammensetzung ist, die mindestens 90 Gew.-% Wasser, bezogen auf das Gesamtgewicht der Zusammensetzung, sowie zumindest ein Tensid und zumindest einen Verdicker enthält, wobei der Anteil an in der Zusammensetzung enthaltenem Verdicker maximal 0,1 Gew.% beträgt, bezogen auf das Gesamtgewicht der Zusammensetzung, und wobei die Viskosität der Zusammensetzung bei 20 °C maximal 12 mPas, insbesondere 8-12 mPas, beträgt.

Der in dem erfindungsgemäßen Verfahren eingesetzte Vliesstoff und das in dem erfindungsgemäßen Verfahren eingesetzte Tränkungsmittel sind so beschaffen wie im Voranstehenden mit Bezug auf das Tränkungsmittel bzw. das Feuchttuch beschrieben.

### Genaue Beschreibung der Erfindung

Die Erfindung wird nun genauer anhand von Ausführungsbeispielen und mit Bezug auf die Figuren beschrieben, die
- in der Figur 1 den Einfluss einer Verdickerkombination aus Konjac Gum und Xanthan Gum auf das Schäumungsverhalten einer Ausführungsform eines erfindungsgemäßen Tränkungsmittels,
- in der Figur 2 den Einfluss einer Verdickerkombination aus Konjac Gum und Xanthan Gum auf das Schäumungsverhalten einer weiteren Ausführungsform eines erfindungsgemäßen Tränkungsmittels, und
- in der Figur 3 den Einfluss von Hydroxyethylcellulose als Verdicker auf das Schäumungsverhalten einer weiteren Ausführungsform eines erfindungsgemäßen Tränkungsmittels zeigen.

### Beispiel 1: Untersuchung des Einflusses des Gehaltes an Verdicker in dem erfindungsgemäßen Tränkungsmittel auf dessen Schäumunqsverhalten

Zur Untersuchung des Einflusses des Gehaltes an Verdicker in einem tensidhaltigen Tränkungsmittel wurden vier Tränkungsmittel hergestellt, die die in den Tabellen 1 bis 4 aufgeführten Zusammensetzungen aufwiesen. Bei den vier Tränkungsmitteln handelte es sich um Tränkungsmittel für Feuchttücher für kosmetische Anwendungen, die jeweils auch Feuchthaltemittel, Puffer, Konservierungsmittel, Lösungsmittel und einen hautpflegenden Wirkstoff enthielten.

Die vier Tränkungsmittel unterschieden sich dabei jeweils durch den Anteil an enthaltenem Verdicker:

Das Tränkungsmittel 1 (erfindungsgemäßes Tränkungsmittel) enthielt 0,04 Gew.-% Konjac Gum und 0,008 Gew.-% Xanthan Gum (vgl. Tabelle 1).

Das Tränkungsmittel 2 (erfindungsgemäßes Tränkungsmittel) enthielt 0,02 Gew.-% Konjac Gum und 0,004 Gew.-% Xanthan Gum (vgl. Tabelle 2).

Das Tränkungsmittel 3 (erfindungsgemäßes Tränkungsmittel) enthielt 0,01 Gew.-% Konjac Gum und 0,002 Gew.-% Xanthan Gum (vgl. Tabelle 3).

Das Tränkungsmittel 4 (nicht erfindungsgemäßes Tränkungsmittel) enthielt weder Konjac Gum noch Xanthan Gum (vgl. Tabelle 4).

Mit Ausnahme des Gehaltes an Verdicker enthielten alle vier Tränkungsmittel die gleichen Zusammensetzungen, wobei ein geringerer Verdicker-Gehalt durch Hinzufügen einer entsprechenden Menge Wasser kompensiert wurde.

**Tabelle 1: Zusammensetzung von Tränkungsmittel 1 (erfindungsgemäßes Tränkungsmittel) mit 0,04 Gew.-% Konjac Gum und 0,008 Gew.-% Xanthan Gum als Verdicker**

| **Inhaltsstoff** | **Anteil** / **[Gew.-%]** |
|---|---|
| Wasser | 97,2330 |
| Glycerin | 1,0325 |
| Citronensäure | 0,4600 |
| Kokosglucosid | 0,4240 |
| Natriumcitrat | 0,3520 |
| Natriumbenzoat | 0,3000 |
| Parfum | 0,1489 |
| Konjac Gum | 0,0400 |
| Xanthan Gum | 0,0080 |
| Dipropylenglycol | 0,0011 |
| Prunus amygdalus dulcis seed extract | 0,0005 |

**Tabelle 2: Zusammensetzung von Tränkungsmittel 2 (erfindungsgemäßes Tränkungsmittel) mit 0,02 Gew.-% Konjac Gum und 0,004 Gew.-% Xanthan Gum als Verdicker**

| **Inhaltsstoff** | **Anteil** / **[Gew.-%]** |
|---|---|
| Wasser | 97,2570 |
| Glycerin | 1,0325 |
| Citronensäure | 0,4600 |
| Kokosglucosid | 0,4240 |
| Natriumcitrat | 0,3520 |
| Natriumbenzoat | 0,3000 |
| Parfum | 0,1489 |
| Konjac Gum | 0,0200 |
| Xanthan Gum | 0,0040 |
| Dipropylenglycol | 0,0011 |
| Prunus amygdalus dulcis seed extract | 0,0005 |

**Tabelle 3: Zusammensetzung von Tränkungsmittel 3 (erfindungsgemäßes Tränkungsmittel) mit 0,01 Gew.-% Konjac Gum und 0,002 Gew.-% Xanthan Gum als Verdicker**

| **Inhaltsstoff** | **Anteil** / **[Gew.-%]** |
|---|---|
| Wasser | 97,2690 |
| Glycerin | 1,0325 |
| Citronensäure | 0,4600 |
| Kokosglucosid | 0,4240 |
| Natriumcitrat | 0,3520 |
| Natriumbenzoat | 0,3000 |
| Parfum | 0,1489 |
| Konjac Gum | 0,0100 |
| Xanthan Gum | 0,0020 |
| Dipropylenglycol | 0,0011 |
| Prunus amygdalus dulcis seed extract | 0,0005 |

**Tabelle 4: Zusammensetzung von Tränkungsmittel 4 (nicht erfindungsgemäßes Tränkungsmittel) ohne einen Gehalt an Verdickern**

| **Inhaltsstoff** | **Anteil** / **[Gew.-%]** |
|---|---|
| Wasser | 97,2810 |
| Glycerin | 1,0325 |
| Citronensäure | 0,4600 |
| Kokosglucosid | 0,4240 |
| Natriumcitrat | 0,3520 |
| Natriumbenzoat | 0,3000 |
| Parfum | 0,1489 |
| Konjac Gum | 0,0000 |
| Xanthan Gum | 0,0000 |
| Dipropylenglycol | 0,0011 |
| Prunus amygdalus dulcis seed extract | 0,0005 |

Von den vier Tränkungsmitteln wurde jeweils die Viskosität gemessen. Dies erfolgte jeweils mit einem DIN-Messsystem 11 gemäß ISO 53019 bei einer Temperatur von 293,15 K und mit einer Umdrehungszahl von 1000 r/min. Die bestimmten Viskositäten sind in der nachstehenden Tabelle 5 angegeben.

**Tabelle 5: Viskositäten der Tränkungsmittel 1, 2, 3 und 4**

| **Tränkungsmittel (TM)** | **Viskosität [mPas]** |
|---|---|
| TM1 | 7 |
| TM2 | 5 |
| TM3 | 4 |
| TM4 | 4 |

Aus den in Tabelle 5 aufgeführten Viskositätswerten ist ersichtlich, dass alle drei erfindungsgemäßen Tränkungsmittel (TM1, TM2, TM3) sowie auch das nicht erfindungsgemäße Tränkungsmittel TM4 eine sehr geringe Viskosität von 4 bis 7 mPas aufwiesen. Dies ist grundsätzlich vorteilhaft für eine Tränkung von Vliesstoff, wobei das jeweilige Tränkungsmittel für eine Eignung zur Tränkung von Vliesstoff zusätzlich auch ein gutes Schäumungsverhalten aufweisen sollte, das Tränkungsmittel sich also durch eine geringe Schaumbildung auszeichnen sollte.

Zur Untersuchung des Schäumungsverhaltens des Tränkungsmittels 1 wurden 60 g des Tränkungsmittels in einen 250 mL Messzylinder überführt. Der Messzylinder wurde mit einem Stopfen verschlossen und anschließend manuell für 30 Sekunden geschüttelt. Dabei sicherte die Hand den Stopfen, während der Unterarm die Schüttelbewegung mit einer Frequenz von 1,5-2 Auf- und AbBewegungen pro Sekunde durchführte. Nach 30 Sekunden Standzeit wurde das Schaumvolumen ermittelt. Dieses ergab sich aus der Höhe des Schaums im Messzylinder abzüglich der Flüssigkeitshöhe.

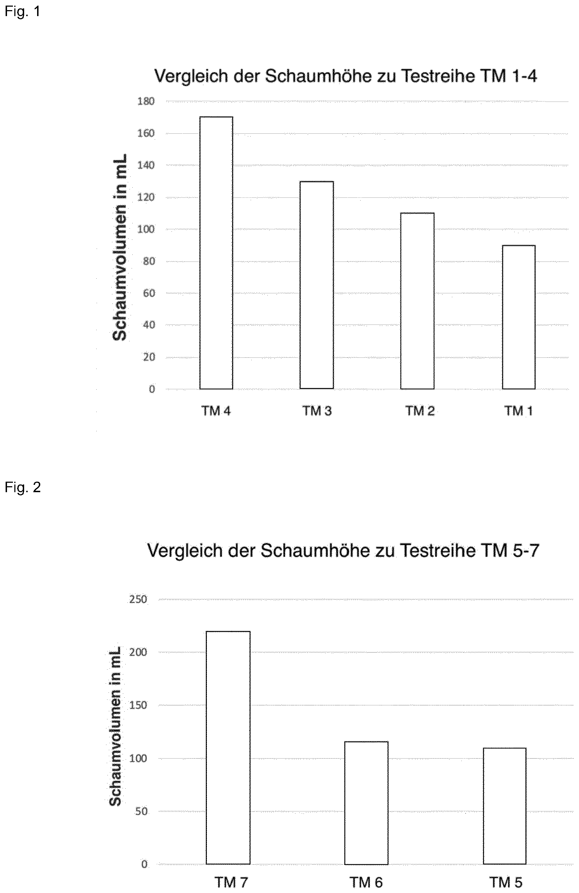

Auf analoge Weise wurde auch das Schäumungsverhalten der Tränkungsmittel 2, 3 und 4 bestimmt. Die Ergebnisse der Untersuchungen sind in der nachstehenden Tabelle 6 zusammengefasst sowie in der Figur 1 graphisch dargestellt.

**Tabelle 6: Zum Vergleich des Schäumungsverhaltens der Tränkungsmittel 1, 2, 3 und 4 ermitteltes Schaumvolumen nach 30 Sekunden Standzeit**

| **Tränkungsmittel (TM)** | **Schaumvolumen [mL]** |
|---|---|
| TM1 | 90 |
| TM2 | 110 |
| TM3 | 130 |
| TM4 | 170 |

Aus den in der Tabelle 6 und in der Figur 1 aufgeführten Ergebnissen wird deutlich, dass der Gehalt an in dem Tränkungsmittel enthaltenem Verdicker sich positiv auf das Schäumungsverhalten des Tränkungsmittels auswirkt, indem dieses deutlich reduziert wird. Dabei ist die Reduzierung des Schäumungsverhaltens bei dem größten Gehalt an Verdicker (in TM 1) am größten und beträgt rund 47 % im Vergleich zu dem entsprechenden Tränkungsmittel ohne einen Gehalt an Verdicker (TM 4). Jedoch auch ein vergleichsweise geringer Gehalt an Verdicker, der (in TM3) insgesamt nur 0,012 % beträgt, bewirkt bereits eine Schaumreduktion um rund 24 % im Vergleich zu dem entsprechenden Tränkungsmittel ohne einen Gehalt an Verdicker (TM 4).

### Beispiel 2: Untersuchung des Einflusses des Gehaltes an Verdicker in einem weiteren erfindungsgemäßen Tränkungsmittel auf dessen Schäumunqsverhalten

Zur weiteren Untersuchung des Einflusses des Gehaltes an Verdicker in einem tensidhaltigen Tränkungsmittel wurden drei weitere Tränkungsmittel hergestellt, die die in den Tabellen 7 bis 9 aufgeführten Zusammensetzungen aufwiesen. Bei den drei Tränkungsmitteln handelte es sich um Tränkungsmittel für Feuchttücher für kosmetische Anwendungen, die neben Tensiden jeweils auch Feuchthaltemittel, Puffer, Konservierungsmittel, Lösungsmittel und mehrere hautpflegende Wirkstoffe enthielten.

Die drei Tränkungsmittel unterschieden sich dabei jeweils durch den Anteil an enthaltenem Verdicker:

Das Tränkungsmittel 5 (erfindungsgemäßes Tränkungsmittel) enthielt 0,04 Gew.-% Konjac Gum und 0,008 Gew.-% Xanthan Gum (vgl. Tabelle 7).

Das Tränkungsmittel 6 (erfindungsgemäßes Tränkungsmittel) enthielt 0,02 Gew.-% Konjac Gum und 0,004 Gew.-% Xanthan Gum (vgl. Tabelle 8).

Das Tränkungsmittel 7 (nicht erfindungsgemäßes Tränkungsmittel) enthielt weder Konjac Gum noch Xanthan Gum (vgl. Tabelle 9).

Mit Ausnahme des Gehaltes an Verdicker enthielten alle drei Tränkungsmittel die gleichen Zusammensetzungen, wobei ein geringerer Verdicker-Gehalt durch Hinzufügen einer entsprechenden Menge Wasser kompensiert wurde.

**Tabelle 7: Zusammensetzung von Tränkungsmittel 5 (erfindungsgemäßes Tränkungsmittel) mit 0,04 Gew.-% Konjac Gum und 0,008 Gew.-% Xanthan Gum als Verdicker**

| **Inhaltsstoff** | **Anteil** / **[Gew.-%]** |
|---|---|
| Wasser | 96,5869 |
| Propylenglycol | 0,8134 |
| Glycerin | 0,7960 |
| Citronensäure | 0,4601 |
| Natriumcitrat | 0,3520 |
| Natriumbenzoat | 0,3004 |
| Cocamidopropyl-Betaine | 0,1600 |
| Parfum | 0,1500 |
| Ethylhexylglycerin | 0,1000 |
| Allantoin | 0,1000 |
| Aloe Barbadensis Leaf Juice | 0,0971 |
| Konjac Gum | 0,0400 |
| Natriumchlorid | 0,0260 |
| Xanthan Gum | 0,0080 |
| Caprylyl/Caprylglucosid | 0,0060 |
| Butylenglycol | 0,0015 |
| Chamomilla Recutita Flower Extract | 0,0015 |
| Glucose | 0,0004 |
| Milchsäure | 0,0004 |
| Kaliumsorbat | 0,0003 |

**Tabelle 8: Zusammensetzung von Tränkungsmittel 6 (erfindungsgemäßes Tränkungsmittel) mit 0,02 Gew.-% Konjac Gum und 0,004 Gew.-% Xanthan Gum als Verdicker**

| **Inhaltsstoff** | **Anteil** / **[Gew.-%]** |
|---|---|
| Wasser | 96,6109 |
| Propylenglycol | 0,8134 |
| Glycerin | 0,7960 |
| Citronensäure | 0,4601 |
| Natriumcitrat | 0,3520 |
| Natriumbenzoat | 0,3004 |
| Cocamidopropyl-Betaine | 0,1600 |
| Parfum | 0,1500 |
| Ethylhexylglycerin | 0,1000 |
| Allantoin | 0,1000 |
| Aloe Barbadensis Leaf Juice | 0,0971 |
| Konjac Gum | 0,0200 |
| Natriumchlorid | 0,0260 |
| Xanthan Gum | 0,0040 |
| Caprylyl/Caprylglucosid | 0,0060 |
| Butylenglycol | 0,0015 |
| Chamomilla Recutita Flower Extract | 0,0015 |
| Glucose | 0,0004 |
| Milchsäure | 0,0004 |
| Kaliumsorbat | 0,0003 |

**Tabelle 9: Zusammensetzung von Tränkungsmittel 7 (nicht erfindungsgemäßes Tränkungsmittel) ohne einen Gehalt an Verdickern**

| **Inhaltsstoff** | **Anteil** / **[Gew.-%]** |
|---|---|
| Wasser | 96,6349 |
| Propylenglycol | 0,8134 |
| Glycerin | 0,7960 |
| Citronensäure | 0,4601 |
| Natriumcitrat | 0,3520 |
| Natriumbenzoat | 0,3004 |
| Cocamidopropyl-Betaine | 0,1600 |
| Parfum | 0,1500 |
| Ethylhexylglycerin | 0,1000 |
| Allantoin | 0,1000 |
| Aloe Barbadensis Leaf Juice | 0,0971 |
| Konjac Gum | 0,0000 |
| Natriumchlorid | 0,0260 |
| Xanthan Gum | 0,0000 |
| Caprylyl/Caprylglucosid | 0,0060 |
| Butylenglycol | 0,0015 |
| Chamomilla Recutita Flower Extract | 0,0015 |
| Glucose | 0,0004 |
| Milchsäure | 0,0004 |
| Kaliumsorbat | 0,0003 |

Zur Untersuchung des Schäumungsverhaltens des Tränkungsmittels 5 wurden 60 g des Tränkungsmittels in einen 250 mL Messzylinder überführt. Der Messzylinder wurde mit einem Stopfen verschlossen und anschließend manuell für 30 Sekunden geschüttelt. Dabei sicherte die Hand den Stopfen, während der Unterarm die Schüttelbewegung mit einer Frequenz von 1,5-2 Auf- und AbBewegungen pro Sekunde durchführte. Nach 30 Sekunden Standzeit wurde das Schaumvolumen ermittelt. Dieses ergab sich aus der Höhe des Schaums im Messzylinder abzüglich der Flüssigkeitshöhe.

Auf analoge Weise wurde auch das Schäumungsverhalten der Tränkungsmittel 6 und 7 bestimmt. Die Ergebnisse der Untersuchungen sind in der nachstehenden Tabelle 10 zusammengefasst sowie in der Figur 2 graphisch dargestellt.

**Tabelle 10: Zum Vergleich des Schäumungsverhaltens der Tränkungsmittel 6, 7 und 8 ermitteltes Schaumvolumen nach 30 Sekunden Standzeit**

| **Tränkungsmittel (TM)** | **Schaumvolumen [mL]** |
|---|---|
| TM5 | 110 |
| TM6 | 116 |
| TM7 | 220 |

Aus den in der Tabelle 10 und in der Figur 2 aufgeführten Ergebnissen wird deutlich, dass der Gehalt an in dem Tränkungsmittel enthaltenem Verdicker sich positiv auf das Schäumungsverhalten des Tränkungsmittels auswirkt, indem dieses deutlich reduziert wird. Dabei ist die Reduzierung des Schäumungsverhaltens bei dem größten Gehalt an Verdicker (in TM 5) am größten und beträgt 50 % im Vergleich zu dem entsprechenden Tränkungsmittel ohne einen Gehalt an Verdicker (TM 7). Jedoch auch ein nur halb so hoher Gehalt an Verdicker, der (in TM 6) insgesamt nur 0,024 % beträgt, bewirkt eine Schaumreduktion um rund 47 % im Vergleich zu dem entsprechenden Tränkungsmittel ohne einen Gehalt an Verdicker (TM 7). Hieraus wird die Schlussfolgerung gezogen, dass nur ein sehr geringer Anteil an Verdicker in dem Tränkungsmittel erforderlich ist, um eine große Schaumreduzierung, beispielsweise um 40-50% zu erreichen. Der Anteil an Verdicker in dem erfindungsgemäßen Tränkungsmittel sollte daher jedenfalls < 0,1 Gew.-% betragen, vorzugsweise < 0,09 Gew.-%, < 0,08 Gew.-%, < 0,07 Gew.-%, < 0,06 Gew.-% oder < 0,05 Gew.-%.

### Beispiel 3: Untersuchung des Einflusses des Gehaltes an Verdicker in einem weiteren erfindungsgemäßen Tränkungsmittel auf dessen Schäumung verhalten

Zur weiteren Untersuchung des Einflusses des Gehaltes an Verdicker in einem tensidhaltigen Tränkungsmittel wurden drei weitere Tränkungsmittel hergestellt, die die in den Tabellen 11 bis 13 aufgeführten Zusammensetzungen aufwiesen. Bei den drei Tränkungsmitteln handelte es sich um Tränkungsmittel für desinfizierende Feuchttücher, die neben Tensiden jeweils auch Puffer, Konservierungsmittel und biozide Wirkstoffe enthielten. Für dieses Beispiel wurden bewusst zwei nicht erfindungsgemäße Tränkungsmittel zum Vergleich hergestellt, nämlich eines, das einen höheren Anteil an Verdicker enthält als erfindungsgemäße Tränkungsmittel, und eines, das gar keinen Anteil an Verdicker enthält.

Die drei Tränkungsmittel unterschieden sich daher jeweils durch den Anteil an enthaltenem Verdicker:

Das Tränkungsmittel 8 (nicht erfindungsgemäßes Tränkungsmittel) enthielt 0,2 Gew.-% Hydroxyethylcellulose (vgl. Tabelle 11).

Das Tränkungsmittel 9 (erfindungsgemäßes Tränkungsmittel) enthielt 0,1 Gew.-% Hydroxyethylcellulose (vgl. Tabelle 12).

Das Tränkungsmittel 10 (nicht erfindungsgemäßes Tränkungsmittel) enthielt keinen Verdicker (vgl. Tabelle 13).

Mit Ausnahme des Gehaltes an Verdicker enthielten alle drei Tränkungsmittel die gleichen Zusammensetzungen, wobei ein geringerer Verdicker-Gehalt durch Hinzufügen einer entsprechenden Menge Wasser kompensiert wurde.

**Tabelle 11: Zusammensetzung von Tränkungsmittel 8 (nicht erfindungsgemäßes Tränkungsmittel) mit 0,2 Gew.-% Hydroxyethylcellulose als Verdicker**

| **Inhaltsstoff** | **Anteil** / **[Gew.-%]** |
|---|---|
| Wasser | 98,1313 |
| Ethanolamin | 0,4080 |
| Benzalkoniumchlorid | 0,3990 |
| Natriumcarbonat | 0,3000 |
| Didecyldimoniumchlorid | 0,2008 |
| Trinatriumdicarboxymethylalaninat | 0,1800 |
| C9-C11 Fettalkoholethoxylat | 0,1513 |
| Alkohol | 0,0251 |
| Natriumhydroxid | 0,0045 |
| Hydroxyethylcellulose | 0,2000 |

**Tabelle 12: Zusammensetzung von Tränkungsmittel 9 (erfindungsgemäßes Tränkungsmittel) mit 0,1 Gew.-% Hydroxyethylcellulose als Verdicker**

| **Inhaltsstoff** | **Anteil** / **[Gew.-%]** |
|---|---|
| Wasser | 98,2313 |
| Ethanolamin | 0,4080 |
| Benzalkoniumchlorid | 0,3990 |
| Natriumcarbonat | 0,3000 |
| Didecyldimoniumchlorid | 0,2008 |
| Trinatriumdicarboxymethylalaninat | 0,1800 |
| C9-C11 Fettalkoholethoxylat | 0,1513 |
| Alkohol | 0,0251 |
| Natriumhydroxid | 0,0045 |
| Hydroxyethylcellulose | 0,1000 |

**Tabelle 13: Zusammensetzung von Tränkungsmittel 10 (nicht erfindungsgemäßes Tränkungsmittel) ohne einen Gehalt an Verdickern**

| **Inhaltsstoff** | **Anteil** / **[Gew.-%]** |
|---|---|
| Wasser | 98,3313 |
| Ethanolamin | 0,4080 |
| Benzalkoniumchlorid | 0,3990 |
| Natriumcarbonat | 0,3000 |
| Didecyldimoniumchlorid | 0,2008 |
| Trinatriumdicarboxymethylalaninat | 0,1800 |
| C9-C11 Fettalkoholethoxylat | 0,1513 |
| Alkohol | 0,0251 |
| Natriumhydroxid | 0,0045 |
| Hydroxyethylcellulose | 0,0000 |

Zur Untersuchung des Schäumungsverhaltens des Tränkungsmittels 8 wurden 60 g des Tränkungsmittels in einen 250 mL Messzylinder überführt. Der Messzylinder wurde mit einem Stopfen verschlossen und anschließend manuell für 30 Sekunden geschüttelt. Dabei sicherte die Hand den Stopfen, während der Unterarm die Schüttelbewegung mit einer Frequenz von 1,5-2 Auf- und AbBewegungen pro Sekunde durchführte. Nach 30 Sekunden Standzeit wurde das Schaumvolumen ermittelt. Dieses ergab sich aus der Höhe des Schaums im Messzylinder abzüglich der Flüssigkeitshöhe.

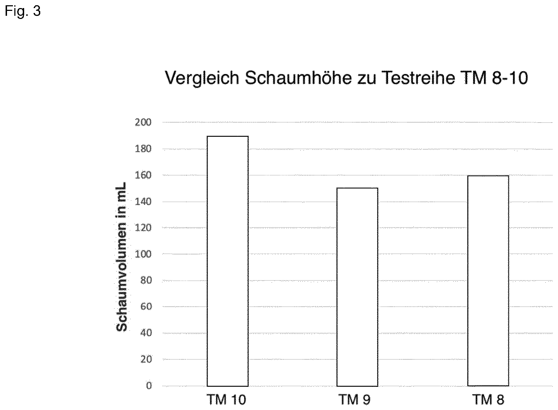

Auf analoge Weise wurde auch das Schäumungsverhalten der Tränkungsmittel 9 und 10 bestimmt. Die Ergebnisse der Untersuchungen sind in der nachstehenden Tabelle 14 zusammengefasst sowie in der Figur 3 graphisch dargestellt.

**Tabelle 14: Zum Vergleich des Schäumungsverhaltens der Tränkungsmittel 8, 9 und 10 ermitteltes Schaumvolumen nach 30 Sekunden Standzeit**

| **Tränkungsmittel (TM)** | **Schaumvolumen [mL]** |
|---|---|
| TM8 | 160 |
| TM9 | 150 |
| TM10 | 190 |

Aus den in der Tabelle 14 und in der Figur 3 aufgeführten Ergebnissen wird deutlich, dass der Gehalt an in dem Tränkungsmittel enthaltenem Verdicker sich positiv auf das Schäumungsverhalten des Tränkungsmittels auswirkt, indem dieses deutlich (im Fall von TM 9 um rund 21 %) reduziert wird. Dagegen bewirkt ein Gehalt an 0,2 % Hydroxyethylcellulose als Verdicker nur eine geringere Reduzierung des Schäumungsverhaltens um rund 16 %.

Nach den Erkenntnissen aus den vorherigen zwei Beispielen ist zu erwarten, dass ein geringerer Gehalt als 0,1 % Verdicker in dem Tränkungsmittel eine noch größere Reduzierung des Schäumungsverhaltens als in TM 9 beobachtet zeigen würde. Für dieses Beispiel wurden jedoch nur verhältnismäßig große Verdickergehalte ausgewählt, um zu zeigen, dass die erfindungsgemäß gefundene Reduzierung des Schäumungsverhaltens von Tränkungsmitteln durch einen Gehalt an Verdickern oberhalb eines Verdickeranteils von 0,1 Gew.-% nicht noch ausgeprägter ist, sondern vielmehr geringer ausfällt.

Die in der vorstehenden Beschreibung, in den Figuren sowie in den Ansprüchen offenbarten Merkmale der Erfindung können sowohl einzeln als auch in beliebigen Kombinationen für die Verwirklichung der Erfindung in ihren verschiedenen Ausführungsformen wesentlich sein.

## Patentansprüche

1. Tränkungsmittel für Feuchttücher, **dadurch gekennzeichnet, dass** das Tränkungsmittel eine wasserbasierte Zusammensetzung ist, die mindestens 90 Gew.-% Wasser, bezogen auf das Gesamtgewicht der Zusammensetzung, sowie zumindest ein Tensid und zumindest einen Verdicker enthält, wobei der Anteil an in der Zusammensetzung enthaltenem Verdicker max. 0,1 Gew.% beträgt, bezogen auf das Gesamtgewicht der Zusammensetzung, **dadurch gekennzeichnet, dass** das zumindest eine Tensid aus der Gruppe ausgewählt ist, die aus Alkylpolyglycosiden, Betain-Tensiden, ethoxylierten Tensiden, Sulfo-Tensiden und quarternären Ammoniumverbindungen besteht, und die Viskosität der Zusammensetzung bei 20 °C maximal 12 mPas beträgt.

2. Tränkungsmittel nach Anspruch 1, **dadurch gekennzeichnet, dass** der zumindest eine Verdicker aus der Gruppe ausgewählt ist, die Konjac Gum, Xanthan Gum, Guar, Cellulose sowie Derivate von Konjac Gum, Xanthan Gum, Guar und Cellulose und auch beliebige Kombinationen dieser umfasst oder daraus besteht.

3. Tränkungsmittel nach einem der Ansprüche 1 oder 2, **dadurch gekennzeichnet, dass** der zumindest eine Verdicker sowie optionale weitere Verdicker aus der Gruppe ausgewählt sind, die aus Konjac Gum, Amorphallus Konjac Root Extract, Xanthan Gum, Dehydroxanthan Gum, Hydroxypropyl Xanthan Gum, Guar, Hydroxypropyl Guar, hydrolisiertem Guar, Carboxymethylhydroxypropyl Guar, Guar Hydroxypropyltrimoniumchlorid, Cellulose, C12-C16 Alkoxy-hydroxypropyl-ethylhydroxyethyl-cellulose, C12-C16 Alkyl-PEG-2-hydroxypropylhydroxyethyl-ethylcellulose, Butoxyhydroxypropylcetyl-hydroxyethylcellulose, Carboxymethyl-hydroxyethylcellulose, Carboxymethyl-celluloseacetatbutyrat, Calciumcarboxymethylcelluose, Celluloseacetatpropionatcarboxylat, Cetylhydroxyethylcellulose, Cellulosesuccinat, Cellulose Gum, Croscarmellose, Ethylcellulose, Hydroxybutylmethylcellulose, hydrolisiertem Cellulose Gum, Hydroxyethylcellulose, Hydroxyethyl-ethylcellulose, Hydroxypropyl-methylcellulose, Hydroxypropylmethylcellulose-stearoxyether, Hydroxypropylcellulose-stearyl-carbamat, Hydroxypropylcellulose, Methyl-Hydroxyethylcellulose, Methyl-ethylcellulose, mikrobieller Cellulose, Methylcellulose, mikrokristalliner Cellulose, oxidierter Cellulose, Natriumstearoxy-PG-hydroxyethylcellulosesulfonat, Natriumsalzen oxidierter Cellulose und Natriumcellulosesulfat besteht.

4. Tränkungsmittel nach einem der voranstehenden Ansprüche, **dadurch gekennzeichnet, dass** das Tränkungsmittel mindestens 95 Gew.-% Wasser enthält und Konjac Gum sowie Xanthan Gum als Verdicker enthält.

5. Tränkungsmittel nach einem der voranstehenden Ansprüche, **dadurch gekennzeichnet, dass** das Tränkungsmittel ein oder mehrere Tenside enthält, die unabhängig voneinander aus der Gruppe ausgewählt sind, die aus Kokosglucosid, Cocamidopropyl-Betainen, Caprylyl/Caprylglucosid, Polysorbat 20, Polysorbat 80, PEG-40 hydriertem Rizinusöl, und C9-C11-Fettalkoholethoxylat, Natriumlaurylsulfat; Natriumlaurylethersulfat, Benz-alkoniumchlorid und Dodecylammoniumchlorid besteht.

6. Tränkungsmittel nach einem der voranstehenden Ansprüche, **dadurch gekennzeichnet, dass** der Anteil an in dem Tränkungsmittel enthaltenen Tensid < 1 Gew.-%, insbesondere < 0,7 Gew.-%, < 0,6 Gew.-% oder < 0,5 Gew.-%, jeweils bezogen auf das Gesamtgewicht der das Tränkungsmittel bildenden Zusammensetzung, beträgt.

7. Tränkungsmittel nach einem der voranstehenden Ansprüche, **dadurch gekennzeichnet, dass** das Tränkungsmittel mindestens 90 Gew.-% Wasser, bevorzugt mindestens 93 Gew.-% Wasser und am meisten bevorzugt mindestens 95 Gew.-% Wasser, jeweils bezogen auf das Gesamtgewicht der das Tränkungsmittel bildenden Zusammensetzung, sowie weiterhin zumindest ein Tensid und eine Kombination aus Konjac Gum sowie Xanthan Gum als Verdicker enthält, wobei der Gesamtanteil an Verdicker in der das Tränkungsmittel bildenden Zusammensetzung maximal 0,1 Gew.% beträgt, bevorzugt weniger als 0,07 Gew.% und besonders bevorzugt weniger als 0,05 Gew.%, jeweils bezogen auf das Gesamtgewicht der Zusammensetzung.

8. Feuchttuch, **dadurch gekennzeichnet, dass** es einen mit einem Tränkungsmittel nach einem der Ansprüche 1 bis 7 getränkten Vliesstoff umfasst oder daraus besteht.

9. Feuchttuch nach Anspruch 8, **dadurch gekennzeichnet, dass** der Vliesstoff aus Viskosefasern und/oder Lyocell-Fasern und/oder PET-Fasern und/oder Hanffasern und/oder Cellulosefasern und/oder Flachsfasern besteht.

10. Feuchttuch nach einem der Ansprüche 8 oder 9, **dadurch gekennzeichnet, dass** der Vliesstoff aus Viskosefasern und/oder Lyocell-Fasern und/oder PET-Fasern und zusätzlich aus 0,1 - 20,0 Gew.-% Mikrofasern und/oder 0,1 - 30,0 Gew.-% Baumwoll-Fasern besteht.

11. Feuchttuch nach einem der voranstehenden Ansprüche, **dadurch gekennzeichnet, dass** der Vliesstoff 30 - 70 Gew.-% Viskosefasern und 30 - 70 Gew.-% PET-Fasern sowie optional 0,1 - 5,0 Gew.-% Mikrofasern und/oder 0,1 - 5,0 Gew.-% Baumwoll-Fasern umfasst oder daraus besteht.

12. Verfahren zur Herstellung eines Feuchttuches, umfassend das Aufbringen eines Tränkungsmittels auf einen Vliesstoff, **dadurch gekennzeichnet, dass** das Tränkungsmittel eine wasserbasierte Zusammensetzung ist, die mindestens 90 Gew.-% Wasser, bezogen auf das Gesamtgewicht der Zusammensetzung, sowie zumindest ein Tensid und zumindest einen Verdicker enthält, wobei der Anteil an in der Zusammensetzung enthaltenem Verdicker maximal 0,1 Gew.% beträgt, bezogen auf das Gesamtgewicht der Zusammensetzung, wobei das zumindest eine Tensid aus der Gruppe ausgewählt ist, die aus Alkylpolyglycosiden, Betain-Tensiden, ethoxylierten Tensiden, Sulfo-Tensiden und quarternären Ammoniumverbindungen besteht, und wobei die Viskosität der Zusammensetzung bei 20 °C maximal 12 mPas beträgt.

13. Verfahren zur Herstellung eines Feuchttuches nach Anspruch 12, **dadurch gekennzeichnet, dass** das Tränkungsmittel, das auf den Vliesstoff aufgebracht wird, einen oder mehrere Verdicker enthält, die aus der Gruppe ausgewählt sind, die aus Konjac Gum, Xanthan Gum, Guar, Cellulose sowie Derivaten von Konjac Gum, Xanthan Gum, Guar und Cellulose sowie beliebigen Kombinationen dieser besteht.

14. Verfahren zur Herstellung eines Feuchttuches nach Anspruch 12 oder 13, **dadurch gekennzeichnet, dass** das Tränkungsmittel, das auf den Vliesstoff aufgebracht wird, ein Tränkungsmittel nach einem der Ansprüche 1 bis 7 ist.

15. Verwendung eines Tränkungsmittels nach einem der Ansprüche 1 bis 7 zur Tränkung von Vliesstoff, wodurch ein Feuchttuch erhalten wird.

## Claims

1. Impregnating agent for wet wipes, **characterized in that** the impregnating agent is a water-based composition containing at least 90 wt.% of water, based on the total weight of the composition, as well as at least one surfactant and at least one thickener, the proportion of thickener contained in the composition being 0.1 wt.% at most, based on the total weight of the composition, **characterized in that** the at least one surfactant is selected from the group consisting of alkylpolyglycosides, betaine surfactants, ethoxylated surfactants, sulfo surfactants and quaternary ammonium compounds, and the viscosity of the composition at 20 °C is 12 mPas at most.

2. Impregnating agent according to claim 1, **characterized in that** the at least one thickener is selected from the group comprising or consisting of konjac gum, xanthan gum, guar, cellulose as well as derivatives of konjac gum, xanthan gum, guar and cellulose and also any combinations thereof.

3. Impregnating agent according to one of the claims 1 or 2, **characterized in that** the at least one thickener and optional further thickeners are selected from the group consisting of konjac gum, amorphophallus konjac root extract, xanthan gum, dehydroxanthan gum, hydroxypropyl xanthan gum, guar, hydroxypropyl guar, hydrolysed guar, carboxymethylhydroxypropyl guar, guar hydroxypropyltrimonium chloride, cellulose, C12-C16 alkoxy-hydroxypropyl-ethylhydroxyethyl-cellulose, C12-C16 alkyl-PEG-2-hydroxypropylhydroxyethyl-ethylcellulose, butoxyhydroxypropylcetyl-hydroxyethylcellulose, carboxymethyl-hydroxyethyl cellulose, carboxymethyl cellulose acetate butyrate, calcium carboxymethyl celluose, cellulose acetate propionate carboxylate, cetyl hydroxyethyl cellulose, cellulose succinate, cellulose gum, croscarmellose, ethyl cellulose, hydroxybutyl methylcellulose, hydrolysed cellulose gum, hydroxyethyl cellulose, hydroxyethyl ethyl cellulose, hydroxypropyl methyl cellulose, hydroxypropyl methyl cellulose stearoxy ether, hydroxypropyl cellulose stearyl carbamate, hydroxypropyl cellulose, methyl hydroxyethylcellulose, methyl ethyl cellulose, microbial cellulose, methyl cellulose, microcrystalline cellulose, oxidized cellulose, sodium stearoxy PG-hydroxyethyl cellulose sulphonate, sodium salts of oxidized cellulose and sodium cellulose sulphate.

4. Impregnating agent according to one of the preceding claims, **characterized in that** the impregnating agent contains at least 95 wt.% of water and contains konjac gum and xanthan gum as thickener.

5. Impregnating agent according to one of the preceding claims, **characterized in that** the impregnating agent contains one or more surfactants which are selected independently of one another from the group consisting of coco glucoside, cocamidopropyl betaines, caprylyl/capryl glucoside, polysorbate 20, polysorbate 80, PEG-40 hydrogenated castor oil, and C9-C11 fatty alcohol ethoxylate, sodium lauryl sulphate, sodium lauryl ether sulphate, benz-alkonium chloride and dodecyl ammonium chloride.

6. Impregnating agent according to one of the preceding claims, **characterized in that** the proportion of the surfactant contained in the impregnating agent is < 1 wt.%, especially < 0.7 wt.%, < 0.6 wt.% or < 0.5 wt.%, in each case based on the total weight of the composition forming the impregnating agent.

7. Impregnating agent according to one of the preceding claims, **characterized in that** the impregnating agent contains at least 90 wt.% of water, preferably at least 93% by weight of water and most preferably at least 95 wt.% of water, in each case based on the total weight of the composition forming the impregnating agent, and furthermore at least one surfactant and a combination of konjac gum and xanthan gum as thickener, the total proportion of thickener in the composition forming the impregnating agent being at most 0.1 wt.%, preferably less than 0.07 wt.% and particularly preferably less than 0.05 wt.%, in each case based on the total weight of the composition.

8. Wet wipe, **characterized in that** it comprises or consists of a nonwoven fabric impregnated with an impregnating agent according to one of claims 1 to 7.

9. Wet wipe according to claim 8, **characterized in that** the nonwoven fabric consists of viscose fibres and/or lyocell fibres and/or PET fibres and/or hemp fibres and/or cellulose fibres and/or flax fibres.

10. Wet wipe according to one of claims 8 or 9, **characterized in that** the nonwoven fabric consists of viscose fibres and/or lyocell fibres and/or PET fibres and additionally of 0.1 - 20.0 wt.% of microfibres and/or 0.1 - 30.0 wt.% of cotton fibres.

11. Wet wipe according to one of the preceding claims, **characterized in that** the nonwoven fabric comprises or consists of 30 - 70 wt.% viscose fibres and 30 - 70 wt.% PET fibres and optionally 0.1 - 5.0 wt.% microfibres and/or 0.1 - 5.0 wt.% cotton fibres.

12. Process for production of a wet wipe comprising applying an impregnating agent to a nonwoven fabric, **characterized in that** the impregnating agent is a water-based composition containing at least 90 wt.% of water, based on the total weight of the composition, as well as at least one surfactant and at least one thickener, the proportion of thickener contained in the composition being at most 0.1 wt.%, based on the total weight of the composition, wherein the at least one surfactant is selected from the group consisting of alkyl polyglycosides, betaine surfactants, ethoxylated surfactants, sulfo-surfactants and quaternary ammonium compounds, and wherein the viscosity of the composition at 20 °C is 12 mPas at most.

13. Process for production of a wet wipe according to claim 12, **characterized in that** the impregnating agent which is applied to the nonwoven fabric contains one or more thickener(s) selected from the group consisting of konjac gum, xanthan gum, guar, cellulose as well as derivatives of konjac gum, xanthan gum, guar and cellulose and any combination thereof.

14. Process for production of a wet wipe according to claim 12 or 13, **characterized in that** the impregnating agent which is applied to the nonwoven fabric is an impregnating agent according to one of claims 1 to 7.

15. Use of an impregnating agent according to any one of claims 1 to 7 for impregnating nonwoven fabric, whereby a wet wipe is obtained.

## Revendications

1. Agent d'imprégnation pour lingettes humides, **caractérisé en ce que** l'agent d'imprégnation est une composition à base d'eau, qui contient au moins 90 % en poids d'eau, par rapport au poids total de la composition, ainsi qu'au moins un tensioactif et au moins un épaississant, la teneur en épaississant contenu dans la composition étant au maximum de 0,1 % en poids, par rapport au poids total de la composition, **caractérisé en ce que** ledit au moins un tensioactif est choisi dans le groupe qui est constitué par les alkylpolyglycosides, tensioactifs bétaïne, tensioactifs éthoxylés, sulfo-tensioactifs et composés d'ammonium quaternaire, et la viscosité de la composition est à 20 °C de 12 mPa.s au maximum.

2. Agent d'imprégnation selon la revendication 1, **caractérisé en ce que** ledit au moins un épaississant est choisi dans le groupe qui comprend ou consiste en la gomme de konjac, la gomme de xanthane, le guar, la cellulose ainsi que les dérivés de gomme de konjac, gomme de xanthane, guar et cellulose et également des associations quelconques de tels épaississants.

3. Agent d'imprégnation selon l'une quelconque des revendications 1 et 2, **caractérisé en ce que** ledit au moins un épaississant ainsi que des épaississants supplémentaire optionnels sont choisis dans le groupe qui est constitué par la gomme de konjac, l'extrait de racines d'*Amorphophallus konjac,* la gomme de xanthane, la gomme de déshydroxanthane, la gomme de xanthane hydroxypropylique, le guar, le guar hydroxypropylique, le guar hydrolysé, le guar carboxyméthylhydroxypropylique, le chlorure d'hydroxypropyltrimonium de guar, la cellulose, une alcoxy(C12-C16)-hy-droxypropyléthylhydroxyéthyl-cellulose, une alkyl(C12-C16)-PEG-2-hydroxypropylhydroxyéthyl-éthylcellulose, la butoxyhydroxypropylcétyl-hydroxyéthylcellulose, la carboxyméthyl-hydroxyéthylcellulose, l'acétate-butyrate de carboxyméthyl-cellulose, la carboxyméthylcelluose calcique, l'acétate-propionate-carboxylate de cellulose, la cétylhydroxyéthylcellulose, le succinate de cellulose, la gomme de cellulose, la croscarmellose, l'éthylcellulose, l'hydroxybutylméthylcellulose, la gomme de cellulose hydrolysée, l'hydroxyéthylcellulose, l'hydroxyéthyl-éthylcellulose, l'hydroxypropyl-méthyl-cellulose, l'hydroxypropylméthylcellulose-stéaroxyéther, le stéaryl-carbamate d'hydroxypropylcellulose, l'hydroxypropylcellulose, la méthyl-hydroxyéthylcellulose, la méthyl-éthylcellulose, la cellulose microbienne, la méthylcellulose, la cellulose microcristalline, la cellulose oxydée, le Sodium Stearoxy-PG-hydroxyethyl Cellulose Sulfonate, les sels sodiques de cellulose oxydée et le sulfate de cellulose sodique.

4. Agent d'imprégnation selon l'une quelconque des revendications précédentes, **caractérisé en ce que** l'agent d'imprégnation contient au moins 95 % en poids d'eau et contient en tant qu'épaississants de la gomme de konjac ainsi que de la gomme de xanthane.

5. Agent d'imprégnation selon l'une quelconque des revendications précédentes, **caractérisé en ce que** l'agent d'imprégnation contient un ou plusieurs tensioactifs qui sont choisis indépendamment les uns des autres dans le groupe qui est constitué par le coco glucoside, les cocosamidopropyl-bétaïnes, le caprylyl/capryl-glucoside, la polysorbate 20, le polysorbate 80, la PEG-40-huile de ricin hydrogénée et un éthoxylate d'alcool gras en C9-C11, le laurylsulfate de sodium ; le lauryléthersulfate de sodium, le chlorure de benzalkonium et le chlorure de dodécylammonium.

6. Agent d'imprégnation selon l'une quelconque des revendications précédentes, **caractérisé en ce que** la teneur en tensioactif contenu dans l'agent d'imprégnation est < 1 % en poids, en particulier < 0,7 % en poids, < 0,6 % en poids ou < 0,5 % en poids, chaque fois par rapport au poids total de la composition constituant l'agent d'imprégnation.

7. Agent d'imprégnation selon l'une quelconque des revendications précédentes, **caractérisé en ce que** l'agent d'imprégnation contient au moins 90 % en poids d'eau, de préférence au moins 93 % en poids d'eau, et de façon tout particulièrement préférée au moins 95 % en poids d'eau, chaque fois par rapport au poids total de la composition constituant l'agent d'imprégnation, ainsi qu'en outre au moins un tensioactif et une association de gomme de konjac et de gomme de xanthane en tant qu'épaississants, la teneur totale en épaississants de la composition constituant l'agent d'imprégnation étant au maximum de 0,1 % en poids, de préférence de moins de 0,07 % en poids et de façon particulièrement préférée de moins de 0,05 % en poids, chaque fois par rapport au poids total de la composition.

8. Lingette humide, **caractérisée en ce qu'**elle comprend ou consiste en un non-tissé imprégné avec un agent d'imprégnation selon l'une quelconque des revendications 1 à 7.

9. Lingette humide selon la revendication 8, **caractérisée en ce que** le non-tissé est constitué de fibres de viscose et/ou fibres de Lyocell et/ou fibres de PET et/ou fibres de chanvre et/ou fibres de cellulose et/ou fibres de lin.

10. Lingette humide selon la revendication 8 ou 9, **caractérisée en ce que** le non-tissé est constitué de fibres de viscose et/ou fibres de Lyocell et/ou fibres de PET et en outre de 0,1 - 20,0 % en poids de microfibres et/ou 0,1 - 30,0 % en poids de fibres de coton.

11. Lingette humide selon l'une quelconque des revendications précédentes, **caractérisée en ce que** le non-tissé comprend ou consiste en 30 - 70 % en poids de fibres de viscose et 30 - 70 % en poids de fibres de PET ainsi qu'en option 0,1 - 5,0 % en poids de microfibres et/ou 0,1 - 5,0 % en poids de fibres de coton.

12. Procédé pour la fabrication d'une lingette humide, comprenant l'application d'un agent d'imprégnation sur un non-tissé, **caractérisé en ce que** l'agent d'imprégnation est une composition à base d'eau, qui contient au moins 90 % en poids d'eau, par rapport au poids total de la composition, ainsi qu'au moins un tensioactif et au moins un épaississant, la teneur en épaississant contenu dans la composition étant au maximum de 0,1 % en poids, par rapport au poids total de la composition, ledit au moins un tensioactif étant choisi dans le groupe qui est constitué par les alkylpolyglycosides, tensioactifs bétaïne, tensioactifs éthoxylés, sulfo-tensioactifs et composés d'ammonium quaternaire, et la viscosité de la composition étant à 20 °C de 12 mPa.s au maximum.

13. Procédé pour la fabrication d'une lingette humide selon la revendication 12, **caractérisé en ce que** l'agent d'imprégnation qui est appliqué sur le non-tissé contient un ou plusieurs épaississants qui sont choisis dans le groupe qui est constitué par la gomme de konjac, la gomme de xanthane, le guar, la cellulose ainsi que les dérivés de gomme de konjac, gomme de xanthane, guar et cellulose et également des associations quelconques de tels épaississants.

14. Procédé pour la fabrication d'une lingette humide selon la revendication 12 ou 13, **caractérisé en ce que** l'agent d'imprégnation qui est appliqué sur le non-tissé est un agent d'imprégnation selon l'une quelconque des revendications 1 à 7.

15. Utilisation d'un agent d'imprégnation selon l'une quelconque des revendications 1 à 7 pour l'imprégnation de non-tissé, ce par quoi une lingette humide est obtenue.
